(19) Europäisches Patentamt

European Patent Office — (11) Veröffentlichungsnummer: **0 140 172 B1**

Office européen des brevets

(12) **EUROPÄISCHE PATENTSCHRIFT**

(45) Veröffentlichungstag der Patentschrift:
07.01.88

(21) Anmeldenummer: 84111560.3

(22) Anmeldetag: 27.09.84

(51) Int. Cl.⁴: **A 61 N 1/05**, B 29 C 71/02,
C 08 J 5/00, C 08 J 7/00,
C 25 B 11/12

(54) Verfahren zur Herstellung von implantierbaren Elektroden aus Glaskohlenstoff.

(30) Priorität: 14.10.83 DE 3337470

(43) Veröffentlichungstag der Anmeldung:
08.05.85 Patentblatt 85/19

(45) Bekanntmachung des Hinweises auf die Patenterteilung:
07.01.88 Patentblatt 88/1

(84) Benannte Vertragsstaaten:
DE FR IT NL SE

(56) Entgegenhaltungen:
EP - A - 0 009 727
EP - A - 0 072 359
DE - A - 2 400 909
DE - A - 2 613 072
DE - A - 2 648 900
DE - A - 2 736 942
US - A - 4 126 924
US - A - 4 409 048

"ULLMANNS ENCYKLOPÄDIE DER TECHNISCHEN CHEMIE", 4. Auflage, Band 14, 1977, "Keramische Farben bis Kork" VERLAG CHEMIE, Weinheim, New York Seite 617

(73) Patentinhaber: Siemens Aktiengesellschaft Berlin und München, Wittelsbacherplatz 2, D-8000 München 2 (DE)

(72) Erfinder: Edeling, Martin, Dr., Weiherstrasse 17,
D-8520 Erlangen-Frauenaurach (DE)
Erfinder: Mund, Konrad, Dr., Langenbrucker Weg 10,
D-8521 Uttenreuth (DE)
Erfinder: Rao, Raghavendra, Dr., Rehweiherstrasse 29a,
D-8520 Erlangen (DE)
Erfinder: Weidlich, Erhard, Dr., Am Tennenbach 41,
D-8521 Spardorf (DE)

## Beschreibung

Die Erfindung betrifft ein Verfahren zur Herstellung von implantierbaren Elektroden aus Glaskohlenstoff mit einem Elektrodenkopf und einem Elektrodenschaft durch Pyrolyse vernetzter Kunstharze.

Elektroden aus Glaskohlenstoff eignen sich als implantierbare Effektoren und Sensoren in der Biomedizin (siehe dazu: DE-OS 26 13 072). Unter Effektoren werden dabei Elektroden verstanden, mit denen eine Reizwirkung ausgeübt wird; Sensoren sind Elektroden, mit denen gemessen wird. Beispiele für Effektoren sind Reizelektroden für Herzschrittmacher sowie Elektroden zur Reizung von Nerven und Muskeln. Als Sensoren kommen Elektroden zur Sauerstoffmessung im Körper in Betracht.

An implantierbare Elektroden, die im allgemeinen aus einem Elektrodenkopf und einem Elektrodenschaft bestehen, werden insbesondere folgende Anforderungen gestellt:
- gute Körperverträglichkeit
- hohe Kapazität und somit geringe Polarisation
- geringer chronischer Reizschwellenanstieg.

Elektroden aus Glaskohlenstoff erfüllen die beiden erstgenannten Anforderungen in hervorragender Weise, sie zeigen jedoch einen langfristigen Reizschwellenanstieg.

Ein möglicher Grund für den bei Glaskohlenstoff-Elektroden zu beobachtenden Reizschwellenanstieg ist die rauhe Oberfläche der Elektroden. Diese Rauhigkeit wird durch eine mechanische Bearbeitung bei der Elektrodenherstellung hervorgerufen. Das bisherige Herstellungsverfahren für Reizelektroden aus Glaskohlenstoff besteht nämlich in einer spanabhebenden Formgebung eines Rohlings aus vernetztem Kunstharz und nachfolgender Pyrolyse des erhaltenen Elektrodenkörpers. Dieses Verfahren ist ausserdem kostenintensiv.

Aufgabe der Erfindung ist es, ein einfaches und kostengünstiges Verfahren anzugeben, das die Herstellung von implantierbaren Elektroden aus Glaskohlenstoff erlaubt, welche die an sie gestellten Forderungen sämtlich erfüllen.

Dies wird erfindungsgemäss dadurch erreicht, dass zunächst der Elektrodenschaft in Form eines Rohlings durch Härten eines Harz-Vorkondensats gefertigt wird, dass an den Rohling in einer Giessform unter Verwendung eines Harz-Vorkondensats der Elektrodenkopf angegossen und vernetzt wird, dass nachfolgend gehärtet wird und dass anschliessend das Kunstharz pyrolytisch in Glaskohlenstoff übergeführt und die auf diese Weise erhaltene Elektrode gegebenenfalls aktiviert wird.

Das erfindungsgemässe Verfahren ermöglicht die Herstellung von Glaskohlenstoff-Elektroden, deran Elektrodenkopf eine glatte Oberfläche, d.h. eine hohe Oberflächengüte, aufweist. Es hat sich nämlich gezeigt, dass nicht nur die Elektrodenrohlinge eine sehr glatte Oberfläche aufweisen, sondern dass diese Oberfläche auch nach der Pyrolyse weitgehend erhalten bleibt. Die glatte Oberfläche bedingt dann ein – im Vergleich zu Elektroden mit rauher Oberfläche – geringeres Bindegewebswachstum, was wiederum einen geringeren langfristigen Reizschwellenanstieg zur Folge hat. Neben der erzielbaren hohen Oberflächengüte der Elektroden sind beim erfindungsgemässen Verfahren auch die niedrigen Investitionskosten von Vorteil.

Das erfindungsgemässe Verfahren zur Herstellung von Glaskohlenstoff-Elektroden beinhaltet ein zweistufiges Giessverfahren. Die Herstellung von Glaskohlenstoff-Formkörpern aus wärmehärtbaren Kunstharzen durch Formen unter Anwendung von Giessverfahren mit nachfolgender Polykondensation und Wärmebehandlung, wobei die Formkörper stufenweise geformt werden, ist zwar im Prinzip bekannt (siehe DE-OS 24 00 909). Beim bekannten Verfahren erfolgt aber ein Schleuderguss, weil nach diesem Verfahren Formkörper hergestellt werden sollen, die Teile mit wenigstens einer Rotationsfläche aufweisen; darüber hinaus dient das bekannte Verfahren zur Herstellung grossdimensionierter Gegenstände. Auf die Herstellung von implantierbaren Elektroden und auf eine hohe Oberflächengüte der hergestellten Formkörper ergeben sich aber keine Hinweise.

Beim erfindungsgemässen Verfahren können vorteilhaft Giessformen aus einem nicht benetzbaren Kunststoff verwendet werden. Als derartige Kunststoffe, die temperaturstabil sein müssen und eine hohe Gebrauchsdauer aufweisen sollen, kommen insbesondere Polypropylen, Polytetrafluorethylen und Silicon in Frage. Der Vorteil von Kunststoff-Giessformen liegt in deren einfacher Herstellung. Bevorzugt werden dabei Giessformen aus Silicon eingesetzt, weil sich bei derartigen Formen die hohe Gasdurchlässigkeit als günstig erweist. Hierbei kann nämlich das bei der Kondensationsreaktion freigesetzte Wasser dampfförmig durch die Giessform entweichen.

Vorzugsweise werden beim erfindungsgemässen Verfahren Giessformen aus Edelstahl, der im allgemeinen gehärtet und hochglanzpoliert ist, eingesetzt. Giessformen aus Edelstahl haben den Vorteil, dass sie praktisch beliebig oft verwendet werden können. Ausserdem können die Elektrodenrohlinge dabei unmittelbar in der Form gehärtet werden, was eine Einsparung an Arbeitsschritten bedeutet. Von Vorteil ist es, wenn die Giessform aus Edelstahl zusätzlich vergoldet wird. Dann kann nämlich auf die Verwendung eines Formtrennmittels verzichtet werden.

Beim erfindungsgemässen Verfahren wird als Harz-Vorkondensat vorzugsweise ein Harz aus vorkondensiertem Furfurylalkohol verwendet, und zwar sowohl zur Herstellung des Elektrodenschaftes als auch des Elektrodenkopfes. Es können aber auch Vorkondensate anderer Harze eingesetzt werden, wie Phenolformaldehyd- und Furanharze. Die Vernetzung der Elektrodenkopfrohlinge erfolgt dabei innerhalb der Giessform, die Härtung kann innerhalb oder ausserhalb der Form vorgenommen werden, wobei die Härtung in der Form bevorzugt wird.

Anhand von Ausführungsbeispielen und einer Figur soll die Erfindung noch näher erläutert werden.

Wird Furfurylalkohol, der preisgünstig und in hoher Reinheit im Handel erhältlich ist, als Ausgangsmaterial für das Kunstharz gewählt, so kann das entsprechende Harz-Vorkondensat daraus durch saure oder basische Katalyse hergestellt werden. Hierzu werden beispielsweise 150 ml Furfurylalkohol bei 50 °C mit 5 ml einer 1%igen Lösung von p-Toluolsulfonsäure in Ethanol versetzt und unter ständigem Rühren innerhalb von 2 h auf eine Temperatur von 90 °C erhitzt, und dann wird das Gemisch für 2 h auf dieser Temperatur gehalten. Dabei wird ein Harz-Vorkondensat erhalten, welches bei dieser Temperatur, d.h. bei 90 °C, eine Viskosität von ca. 50 mPa·s besitzt und somit noch ausreichend dünnflüssig ist, um beispielsweise in 1 mm weite Kapillaren eingespritzt werden zu können. Ein derartiges Vorkondensat ist im übrigen bei Raumtemperatur mehrere Wochen lang lagerfähig.

Neben p-Toluolsulfonsäure können auch andere (starke) organische Säuren als Härtungskatalysatoren eingesetzt werden, beispielsweise andere aromatische Sulfonsäuren, Trichloressigsäure und Oxalsäure. Die Katalysatorkonzentration beträgt im allgemeinen 0,01 bis 1 Gew.-%, bezogen auf das eingesetzte Ausgangsmaterial (zur Herstellung des Kunstharzes). Die Temperatur bei der Herstellung des Harz-Vorkondensats liegt im allgemeinen im Bereich zwischen 50 und 120 °C, die Zeitdauer beträgt dabei zwischen 2 und 16 h.

Die Herstellung der Elektrodenrohlinge erfolgt, wie bereits ausgeführt, nach einem zweistufigen Giessverfahren, wobei zunächst der Elektrodenschaft gefertigt wird. Dazu wird Harz-Vorkondensat in einen Siliconschlauch, beispielsweise mit einem Innendurchmesser von 2,4 mm und einer Länge bis zu 50 cm, eingebracht. Das Harz-Vorkondensat wird dann – im Siliconschlauch – bei einer Temperatur von 70 bis 100 °C innerhalb 24 bis 48 h vernetzt und nachfolgend bei 150 bis 200 °C innerhalb von 24 h vollends gehärtet. Von dem dabei erhaltenen Stab aus Kunstharz wird der Siliconschlauch entfernt und der Stab in Stücke mit einer geeigneten Länge, beispielsweise von ca. 4 mm (Durchmesser: 2,4 mm), zerschnitten, welche Elektrodenschaftrohlinge darstellen.

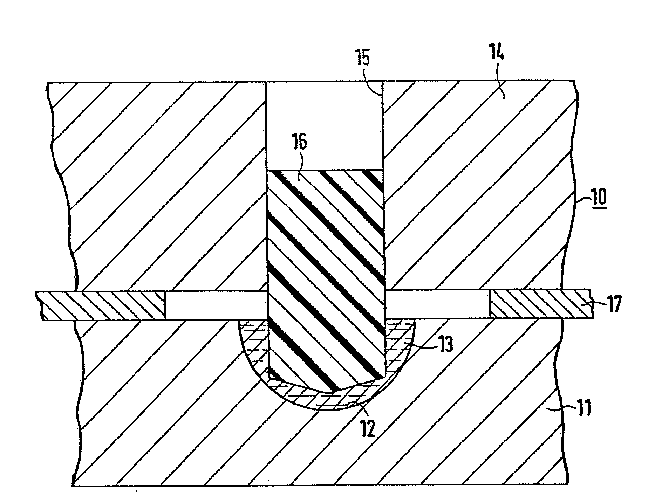

Zur Herstellung der Elektrodenrohlinge dient, wie in der Figur dargestellt, eine zweiteilige Giessform 10. Das Unterteil 11 dieser Giessform weist eine halbkugelförmige Aussparung 12 in Form des Elektrodenkopfes auf. Diese Aussparung wird teilweise mit Harz-Vorkondensat 13 gefüllt. Das Oberteil 14 der Giessform besitzt eine Bohrung 15, welche zur Aufnahme des Elektrodenschaftrohlings 16 dient. Das Oberteil 14 wird – unter Verwendung eines Abstandhalters 17 – in einem Abstand von 0,5 bis 1,5 mm auf das Unterteil 11 aufgesetzt, und dann wird der Elektrodenschaftrohling 16 in der Weise in die Bohrung 15 eingeführt, dass er nahezu bis auf den Grund der Aussparung 12 reicht. Wird die Unterseite des Rohlings 16, wie aus der Figur ersichtlich, leicht angespitzt, so wird der Elektrodenschaft vom flüssigen Harz-Vorkondensat 13 gleichmässig und blasenfrei benetzt. Die Menge des Harz-Vorkondensats wird im übrigen so gewählt, dass nach dem Einbringen des Rohlings die halbkugelförmige Aussparung vollständig ausgefüllt ist.

Das Harz-Vorkondensat, das in die erwärmte Form eingebracht wird, wird dann innerhalb von 24 bis 28 h bei stetiger Erwärmung von einer Temperatur von 70 °C auf 100 °C vernetzt. Anschliessend wird der noch flexible Elektrodenrohling – bei einer Temperatur von 150 bis 200 °C (Dauer: 24 h) – an der Luft gehärtet, was innerhalb oder ausserhalb der Giessform erfolgen kann.

Das vorstehend beschriebene zweistufige Giessverfahren bietet gegenüber einem Einstufenverfahren, bei dem der Elektrodenschaft und der Elektrodenkopf gleichzeitig gegossen werden, folgenden Vorteil. Beim Einstufenverfahren erweist sich die Abdichtung der beiden Formteile gegeneinander als problematisch. Hierbei dringen nämlich stets Spuren des Harz-Vorkondensats in den Kapillarspalt zwischen dem Oberteil und dem Unterteil ein, so dass Hohlräume am Übergang vom Elektrodenkopf zum Elektrodenschaft unvermeidlich sind. Dieses Problem tritt beim Zweistufenverfahren nicht auf.

Die Überführung der Elektrodenrohlinge in die eigentlichen Elektroden erfolgt durch Pyrolyse, wobei aus dem vernetzten Kunstharz Glaskohlenstoff gebildet wird. Die Pyrolyse wird unter inerter Atmosphäre vorgenommen, wobei als Schutzgas beispielsweise Stickstoff oder Argon dient. Die Pyrolysetemperatur liegt im allgemeinen zwischen 700 und 2000 °C und beträgt vorzugsweise ca. 1100 °C. Dabei wird mit einer Temperaturrate von 40 °C/h auf die Pyrolysetemperatur aufgeheizt und die Endtemperatur für 5 h gehalten. Nach der Pyrolyse, die im allgemeinen in einem Rohrofen erfolgt, wird mit 50 °C/h abgekühlt. Im übrigen bestimmt der Elektrodenrohling dann bereits die endgültige Form der Elektrode, wenn der während der Pyrolyse erfolgende isotrope Schwund des Materials von linear ca. 20% beachtet wird.

An den Herstellungsprozess der Elektroden kann sich noch eine Aktivierung anschliessen. Hierbei werden die Elektroden, im Anschluss an die Pyrolyse, beispielsweise an Luft auf eine Temperatur von 450 bis 500 °C erhitzt (Dauer: ca. 1 h). Durch die Aktivierung wird eine Oberfläche mit mikroporöser Struktur erhalten, was sich auf die elektrischen Eigenschaften der Elektroden sehr vorteilhaft auswirkt.

Die beim erfindungsgemässen Verfahren hergestellten Elektrodenrohlinge (Grünlinge) weisen eine vollkommen glatte, homogene Oberfläche auf, was beispielsweise aus rasterelektronenmikroskopischen Aufnahmen (REM-Aufnahmen) klar ersichtlich ist. Diese Eigenschaft bleibt bei der Pyrolyse weitgehend erhalten, und auch nach einer Luftaktivierung zeigen sich in REM-Aufnahmen keine sichtbaren Veränderungen der Oberflächenstruktur.

An den nach dem erfindungsgemässen Verfahren hergestellten Elektroden werden – in physiologischer Kochsalzlösung (pH = 7) – Kapazitäten von 5 mF bei 1 Hz bzw. 100 µF bei 1 kHz ermittelt. Diese Kapazitäten sind für Implantationszwecke ausreichend. Bei einer Elektrodenfläche von 0,1 cm$^2$ erreicht die Flächenkapazität – bei 1 Hz – somit einen Wert von 50 mF · cm$^{-2}$, was für eine sehr feine, gleichmässige Mikroporosität spricht.

## Patentansprüche

1. Verfahren zur Herstellung von implantierbaren Elektroden aus Glaskohlenstoff mit einem Elektrodenkopf und einem Elektrodenschaft durch Pyrolyse vernetzter Kunstharze, dadurch gekennzeichnet, dass zunächst der Elektrodenschaft in Form eines Rohlings durch Härten eines Harz-Vorkondensats gefertigt wird, dass an den Rohling in einer Giessform unter Verwendung eines Harz-Vorkondensats der Elektrodenkopf angegossen und vernetzt wird, dass nachfolgend gehärtet wird, und dass anschliessend das Kunstharz pyrolytisch in Glaskohlenstoff übergeführt und die auf diese Weise erhaltene Elektrode gegebenenfalls aktiviert wird.

2. Verfahren nach Anspruch 1, dadurch gekennzeichnet, dass eine Giessform aus einem nicht benetzbaren Kunststoff, insbesondere Silicon, verwendet wird.

3. Verfahren nach Anspruch 1, dadurch gekennzeichnet, dass eine Giessform aus gegebenenfalls vergoldetem Edelstahl verwendet wird.

4. Verfahren nach einem der Ansprüche 1 bis 3, dadurch gekennzeichnet, dass ein Harz aus vorkondensiertem Furfurylalkohol verwendet wird.

## Revendications

1. Procédé de fabrication d'électrodes susceptibles d'être implantées en carbone vitreux, ayant une tête d'électrode et une tige d'électrode, par pyrolyse de résines réticulées de matières plastiques, caractérisé en ce qu'il consiste à préparer d'abord la tige d'électrode sous la forme d'une ébauche, par durcissement d'un précondensat de résine, à mouler et à réticuler la tête d'électrode sur l'ébauche dans un moule de coulée en utilisant un précondensat de résine, à la durcir ensuite et à transformer ensuite la résine de matière plastique pyrolytiquement en carbone vitreux et à activer, le cas échéant, l'électrode ainsi obtenue.

2. Procédé suivant la revendication 1, caractérisé en ce qu'il consiste à utiliser un moule de coulée constitué d'une matière plastique non mouillable, notamment en silicone.

3. Procédé suivant la revendication 1, caractérisé en ce qu'il consiste à utiliser un moule de coulée en acier fin, le cas échéant revêtu d'or.

4. Procédé suivant l'une des revendications 1 à 3, caractérisé en ce qu'il consiste à utiliser une résine en alcool furfurylique précondensé.

## Claims

1. A process for the production of implantable electrodes made of glassy carbon and comprising an electrode head and an electrode shaft, by the pyrolysis of cross-linked synthetic resins, characterised in that the electrode shaft is first produced in the form of a blank by hardening a resin-precondensate; that the electrode head is cast onto the blank in a casting mould using a resin-precondensate and cross-linked; that hardening is then carried out; and that finally, the synthetic resin is pyrolytically converted into glassy carbon and the electrode so obtained is activated if necessary.

2. A process as claimed in Claim 1, characterised in that a casting mould is used, made of a non-wettable synthetic material, in particular, silicon.

3. A process as claimed in Claim 1, characterised in that a casting mould is used, which is made of high-grade steel, which, if necessary, is gilded.

4. A process as claimed in one of Claims 1 to 3, characterised in that a resin consisting of precondensed furfuryl alcohol is used.